(19) Europäisches Patentamt
European Patent Office
Office européen des brevets

(11) EP 1 078 914 A1

(12) **EUROPÄISCHE PATENTANMELDUNG**

(43) Veröffentlichungstag:
**28.02.2001 Patentblatt 2001/09**

(51) Int. Cl.[7]: **C07C 205/12**, C07C 201/12

(21) Anmeldenummer: **00117274.1**

(22) Anmeldetag: **16.08.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **27.08.1999 DE 19940861**

(71) Anmelder: **BAYER AG**
**51368 Leverkusen (DE)**

(72) Erfinder:
- **Mais, Franz-Josef, Dr.**
  **40591 Düsseldorf (DE)**
- **Lahr, Helmut, Dr.**
  **51519 Odenthal (DE)**

(54) **Verfahren zur Herstellung von 2-Chlor-4-nitroalkylbenzol**

(57) Es wird ein Verfahren zur Herstellung von 2-Chlor-4-nitroalkylbenzolen bereitgestellt, das eine Kernchlorierung von 4-Nitroalkylbenzolen mit elementarem Chlor oder Chlor abgebenden Verbindungen in flüssiger Phase in Gegenwart von Friedel-Crafts-Katalysatoren und speziellen schwefelhaltigen aromatischen Verbindungen als Co-Katalysatoren umfasst.

EP 1 078 914 A1

## Beschreibung

Die Erfindung betrifft ein Verfahren zur Herstellung von 2-Chlor-4-nitroalkylbenzolen durch Kernchlorierung von 4-Nitroalkylbenzolen mit elementarem Chlor oder Chlor abgebenden Verbindungen in flüssiger Phase in Gegenwart von Friedel-Crafts-Katalysatoren und schwefelhaltigen aromatischen Verbindungen als Co-Katalysatoren.

Die Umsetzung von 4-Nitrotoluol mit Chlor in Gegenwart von Lewis-Säure-Katalysatoren wie $FeCl_3$ (US-A-3,341,595; J. Chem. Soc. 1927, 2905) oder $SbCl_3$ (Bull. Soc. Chim. Belg. 61 (1952) 317) ist bekannt. Als Hauptprodukt entsteht dabei das gewünschte 2-Chlor-4-nitrotoluol. Dieses Ergebnis lässt sich auch mit Hilfe der allgemein gültigen Substituentenregeln für die elektrophile aromatische Substitution herleiten. Beispielsweise zeigen die Lehrbücher Streitwieser, Jr. and Heathcock „Introduction to Organic Chemistry", 1985, 669 — 670 (Mac Millan Publishing Co.) und Mc Murry „Organic Chemistry", 1985, 506 — 507 (Brooks-Cole Publishing Co.), dass die Wirkung der Methylgruppe und der dazu para-ständigen Nitrogruppe additiv ist und die bei weitem bevorzugte Position für den Eintritt des dritten Substituenten die ortho-Stellung zur Methylgruppe ist. Als Nebenprodukte werden jedoch auch höherchlorierte, stellungsisomere Dichlor-4-nitrotoluole und 4-Nitrobenzylchlorid sowie die davon abgeleiteten Kernchlorderivate beobachtet.

Aus den erwähnten allgemeinen Substitutionsregeln lassen sich jedoch keine Erkenntnisse zur Stufenselektivität bei der elektrophilen aromatischen Substitution ableiten. Unter der Stufenselektivität versteht man z.B. im vorliegenden Fall für die Kernchlorierung von 4-Nitroalkylbenzolen, wie hoch der Anteil der gewünschten Monochlorstufe im Reaktionsgemisch ist bezogen auf den Umsatz an 4-Nitroalkylbenzol. Üblicherweise verlaufen derartige Chlorierungen zu Beginn der Umsetzung, wenn noch praktisch reines Ausgangsmaterial vorliegt, mit hoher Selektivität. Entsprechend den genannten Substituentenregeln entsteht somit nahezu ausschließlich 2-Chlor-4-nitroalkylbenzol. Im Laufe der Chlorierung verarmt das Reaktionsgemisch jedoch an 4-Nitroalkylbenzol, und gleichzeitig steigt sein Gehalt an 3-Chlor-4-nitroalkylbenzol. Das führt ferner dazu, dass auch das gewünschte Produkt eine Chlorierung erleidet und zu unbrauchbaren Dichlornitroalkylbenzolen abreagiert.

Eigene Untersuchungen zur Kernchlorierung von 4-Nitrotoluol mit $FeCl_3$ als Katalysator haben ergeben, dass die Stufenselektivität noch nicht ausreichend ist. Der maximale, im Reaktionsgemisch erreichbare Anteil an 2-Chlor-4-nitrotoluol liegt knapp unter 90 %. Dieses Ergebnis stimmt gut mit den Angaben aus RO 76842 (CA 100: 8516 (1984)) überein. Dort ist für Chloriergemische von 4-Nitrotoluol ein Gehalt von 86 — 89 % an 2-Chlor-4-nitrotoluol angegeben.

Es ist ferner bekannt, dass die Stufenselektivität der Chlorierung von 4-Nitroalkylbenzolen durch zusätzliche Co-Katalysatoren gesteigert werden kann.

Als Co-Katalysator kann man beispielweise Jod in kleinen Mengen einsetzen (Naturwiss. 17 (1929) 13, Houben-Weyl „Methoden der organischen Chemie" Band V/3 (1962), 704, JP-B-75/7589 (CA 83: 113927 (1975)), CS 193662 (1984)). Zusätzlich ist in US-A-3,005,031 beschrieben, dass bei Verwendung von feuchtem 4-Nitrotoluol in Gegenwart von Eisen, Jod und $PCl_3$ chloriert werden kann. Eigene Untersuchungen zum Einfluss von Jod auf die Stufenselektivität haben gezeigt, dass unter diesen Bedingungen ein Gehalt von bis zu 95 % 2-Chlor-4-nitrotoluol im Reaktionsgemisch erreichbar ist.

Das Verfahren unter Zusatz von Jod hat jedoch erhebliche Nachteile, so dass es technisch nicht brauchbar ist. Vor allem verbleibt nahezu das gesamte Jod selbst nach mehreren wässrigen Wäschen im Chloriergemisch, so dass auch nach der katalytischen Hydrierung und Destillation ein mit Jod verunreinigtes 3-Chlor-4-alkylanilin anfällt. Ein derart verunreinigtes Material kann beispielsweise für eine Phosgenierung zum 3-Chlor-4-alkylphenyl-isocyanat nicht verwendet werden.

Auch aus der DE-OS-31 28 442 ist die Chlorierung von 4-Nitrotoluol mit Jod als alleinigem Katalysator bekannt. Die Chlorierung wird bei einer Temperatur zwischen dem Schmelzpunkt des 4-Nitrotoluols und 120°C durchgeführt. Eingesetzt werden dabei 0,1-10 Gew.% Jod, bezogen auf 4-Nitrotoluol. Auch dieses Verfahren liefert hoch jodhaltige Chlorierungsgemische, die in der Praxis nicht brauchbar sind. Außerdem entweicht bei diesem Verfahren ein Großteil des eingeleiteten Chlors ungenutzt aus der Reaktionsmischung, so dass beispielsweise zwecks Erreichung eines industriell erforderlichen Umsatzes von mehr als 90 mol-%, Chlor bis zu 300 mol-% Chlor pro Mol 4-Nitrotoluol eingeleitet werden müssen.

Auch in der EP-A-0 399 293 wird die Monokernchlorierung von 2-Chlor-4-nitroalkylbenzolen beschrieben. Hierbei werden heterocyclische 5- oder 6-gliedrige dibenzokondensierte Verbindungen, die mindestens ein Schwefelatom enthalten, als Co-Katalysatoren eingesetzt. Als dibenzokondensierte Schwefelheterocyclen finden z.B. Verbindungen aus den Klassen der Phenoxathiine, der Thianthrene, der Thianthren-5-oxide, der Thianthren-5,5-dioxide oder der Dibenzothiophene Anwendung. Auf diesem Weg können gute Selektivitäten erzielt werden. Es werden beispielsweise Gehalte von 95 — 96 % 2-Chlor-4-nitrotoluol im Chloriergemisch erreicht. Aber auch dieses Verfahren hat erhebliche Nachteile, die eine Anwendung in der großtechnischen Praxis nicht erlauben. Zum Beispiel müssen die Co-Katalysatoren über relativ aufwendige Verfahren synthetisiert werden, da sie kommerziell nicht erhältlich sind. Weiterhin bestehen gegen die Verbindungen aus den genannten Klassen erhebliche Bedenken aus toxikologischer Sicht.

Aufgabe der vorliegenden Erfindung war es daher, ein technisch anwendbares Verfahren zur Kernchlorierung von 4-Nitroalkylbenzolen zur Verfügung zu stellen, welches eine erhöhte Stufenselektivität aufweist. Dieses Verfahren soll insbesondere bei hohen Chlorierungsgraden, wenn der Anteil an 4-Nitroalkylbenzol im Reaktionsgemisch nur noch sehr klein ist, dafür sorgen, dass der sich bildende Anteil an höher chlorierten Produkten gering ist. Eine derart gewünschte Erhöhung der Stufenselektivität ist gleichbedeutend mit einer Steigerung der Ausbeute des Zielprodukts 2-Chlor-4-nitroalkylbenzol.

Gegenstand der vorliegenden Erfindung ist ein Verfahren zur Herstellung von 2-Chlor-4-nitroalkylbenzolen der Formel I,

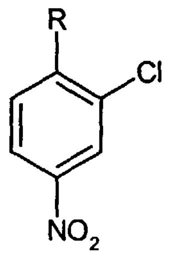

(I)

in der R für geradkettiges oder verzweigtes $C_1$ — $C_4$ Alkyl steht,
durch Kernchlorierung des zugrundeliegenden 4-Nitroalkylbenzols mit elementarem Chlor oder Chlor abgebenden Verbindungen in flüssiger Phase in Gegenwart eines Friedel-Crafts-Katalysators und eines schwefelhaltigen aromatischen Co-Katalysators, dadurch gekennzeichnet, dass als schwefelhaltiger aromatischer Co-Katalysator Diarylsulfide der Formel II

$$Ar^1 — S — Ar^2 \qquad (II)$$

eingesetzt werden, worin
$Ar^1$ und $Ar^2$ unabhängig voneinander Phenyl oder ein- oder mehrfach durch geradkettiges oder verzweigtes $C_1$-$C_8$-Alkyl, geradkettiges oder verzweigtes $C_1$-$C_8$-Halogenalkyl, geradkettiges oder verzweigtes $C_1$-$C_8$-Alkoxy, geradkettiges oder verzweigtes $C_1$-$C_8$-Alkylmercapto, Halogen, Cyano, Nitro, Hydroxy, Phenylmercapto oder Phenyl substituiertes Phenyl oder Naphthyl bedeuten.

Das erfindungsgemäße Verfahren der Kernchlorierung zeichnet sich durch eine exzellente Stufenselektivität aus. Beispiele für die erfindungsgemäß in 2-Position einfach zu chlorierenden Verbindungen sind 4-Nitrotoluol, 4-Nitroethylbenzol, 4-Nitropropylbenzol und 4-Nitro-n-butylbenzol. Besonders geeignet ist das erfindungsgemäße Verfahren für die Chlorierung von 4-Nitrotoluol.

Bevorzugt werden Co-Katalysatoren eingesetzt, bei denen $Ar^1$ und $A^2$ Phenyl oder ein bis dreifach durch Fluor, Chlor oder Brom, geradkettiges oder verzweigtes $C_1$-$C_4$-Alkyl, $C_1$-$C_4$-Halogenalkyl, $C_1$-$C_4$-Alkylmercapto oder Phenyl substituiertes Phenyl bedeuten.

Besonders bevorzugt werden Co-Katalysatoren eingesetzt, bei denen $Ar^1$ und $Ar^2$ Phenyl oder ein oder zweifach durch Chlor, Brom oder Methyl substituiertes Phenyl bedeuten.

Insbesondere bewährt hat sich die Verwendung von Diphenylsulfid, 4,4'-Dibromdiphenylsulfid, 4-Chlor-4'-trifluormethyldiphenylsulfid, 4,4'-Dicyanodi-phenylsulfid und 4,4'-Dihydroxydiphenylsulfid.

Es ist darüber hinaus auch möglich, Gemische von zwei oder mehr erfindungsgemäßen Co-Katalysatoren einzusetzen.

Die erfindungsgemäß einzusetzenden Diarylsulfide der Formel II, wie z.B. Diphenylsulfid, sind entweder kommerziell erhältlich oder können nach zahlreichen, an sich bekannten Methoden hergestellt werden. Möglich ist z.B. die Umsetzung von Arylhalogeniden mit Schwefelwasserstoff oder dessen Salzen oder mit Arylmercaptanen oder dessen Salzen (siehe z.B. H. J. Cristau et al., Synthesis 1981, 892) oder die Umsetzung von Schwefelhalogeniden oder Arylsulfonylhalogeniden oder Cyaniden mit Aromaten (siehe z.B. T. R. Forbus et al. J. Org. Chem. 1979 (44), 313).

Das erfindungsgemäße Verfahren wird in flüssiger Phase durchgeführt, wobei das 4-Nitroalkylbenzol der Formel I in flüssiger (geschmolzener) Form oder gegebenenfalls in Verdünnung mit einem inerten Lösungsmittel eingesetzt wird. Geeignete Lösungsmittel sind solche, die durch Chlor bzw. durch das Katalysatorsystem unter den Bedingungen des erfindungsgemäßen Verfahrens nicht angegriffen werden. Solche Lösungsmittel sind dem Fachmann grundsätzlich bekannt und umfassen beispielsweise Methylenchlorid, Chloroform, Tetrachlorkohlenstoff und Essigsäure. In bevorzugter Weise wird ohne Lösungsmittel gearbeitet.

Als Chlorierungsmittel für das erfindungsgemäße Verfahren wird vorzugsweise Chlor eingesetzt, das flüssig

oder gasförmig in das Reaktionsgemisch eingeleitet werden kann; bevorzugt wird gasförmiges Chlor eingeleitet. Es können jedoch auch andere Chlorierungsmittel verwendet werden, die unter den Reaktionsbedingungen Chlor abgeben, wie beispielsweise Sulfurylchlorid.

Das erfindungsgemäße Verfahren kann grundsätzlich bei einer Temperatur vom Erstarrungspunkt bis zum Siedepunkt des Reaktionsgemisches durchgeführt werden. Im allgemeinen liegt die Reaktionstemperatur bei 50 — 150°C, bevorzugt bei 70 — 120°C und besonders bevorzugt bei 80 — 100°C. Der Reaktionsdruck kann normal, vermindert oder erhöht sein und ist grundsätzlich unkritisch. Die Durchführung bei Normaldruck ist wegen der Vereinfachung der Reaktionsapparatur bevorzugt. Eine Erhöhung des Druckes kann dann vorteilhaft sein, wenn ein niedrigsiedendes Reaktionsgemisch mit einem niedrigsiedenden Lösungsmittel in der flüssigen Phase gehalten werden soll. In diesem Fall wird in bevorzugter Weise unter dem sich automatisch einstellenden Eigendruck eines solchen Reaktionsgemisches gearbeitet.

Das erfindungsgemäße Verfahren ermöglicht die selektive Herstellung eines 2-Chlor-4-nitroalkylbenzols, d.h. einer monochlorierten Verbindung. Für eine solche Monochlorierung werden 90 — 130 Mol-%, bevorzugt 95 — 110 Mol-% Chlor in elementarer Form oder in Form einer Chlor abgebenden Substanz pro Mol 4-Nitroalkylbenzol eingesetzt.

Ein geringer Wassergehalt der Reaktionsmischung ist im allgemeinen unkritisch. In bevorzugter Weise können daher alle Einsatzstoffe in nicht speziell getrockneter Form verwendet werden. Selbstverständlich ist aber eine solche vollständige Trocknung einzelner oder aller Einsatzstoffe ebenfalls möglich. Unter einem geringen Wassergehalt wird ein solcher verstanden, der nicht über den Sättigungsgrenzen der jeweiligen Einsatzstoffe liegt. Ferner darf ein solch geringer Wassergehalt nicht so groß sein, dass der eingesetzte Friedel-Crafts-Katalysator durch Hydrolyse vollständig aufgebraucht wird. Möglich sind beispielsweise geringe Wassergehalte im Reaktionsgemisch bis 250 ppm, bevorzugt bis 150 ppm.

Friedel-Crafts-Katalysatoren für das erfindungsgemäße Verfahren sind alle dem Fachmann bekannten Friedel-Crafts-Katalysatoren, wie Antimonchloride, Aluminiumchlorid oder Eisenchloride. Es können jedoch auch Elemente oder Elementverbindungen eingesetzt werden, die während der Chlorierung einen Friedel-Crafts-Katalysator (Lewis-Säure) bilden. Hierzu gehören beispielsweise die Elemente Eisen, Antimon, Aluminium oder Zink, sowie die Oxide, Sulfide oder Carbonyle dieser Elemente, ferner Salze schwacher Säuren, wie die Carbonate. Beispielsweise können Antimonoxide, Eisenoxide, Eisensulfide, Eisencarbonyle oder Eisencarbonate eingesetzt werden. Anstelle der erwähnten Chloride können auch die Bromide, gegebenenfalls auch die Fluoride der genannten Elemente eingesetzt werden. Bevorzugte Friedel-Crafts-Katalysatoren sind Antimonchloride und Eisenchloride, besonders bevorzugt ist Eisen-(III)-chlorid.

Die Einsatzmengen des Friedel-Crafts-Katalysators oder eines Gemisches mehrerer von ihnen können in weiten Grenzen variiert werden. So ist bereits bei einem Zusatz von 0,005 Gew.-% eine Katalysatorwirkung erkennbar. Andererseits können auch 10 Gew.-% oder mehr des Friedel-Crafts-Katalysators zugesetzt werden. So hohe Mengen bieten jedoch im allgemeinen keinen Vorteil, sind aber kostenintensiv und führen gegebenenfalls bei der Aufarbeitung zu Schwierigkeiten. Üblicherweise wird der Friedel-Crafts-Katalysator oder ein Gemisch mehrerer von ihnen in einer Menge von 0,01 — 3 Gew.-%, bevorzugt von 0,05 — 1,5 Gew.-%, besonders bevorzugt von 0,1 — 1,00 Gew.-%, eingesetzt, jeweils bezogen auf die Menge des eingesetzten 4-Nitro-alkylbenzols.

Die erfindungsgemäße Co-Katalysatoren oder Gemische mehrerer von ihnen können in einer in weiten Grenzen variierbaren Menge eingesetzt werden. Bei Mengen unter 0,01 Gew.-% lässt jedoch die Co-katalytische Wirkung nach. Mengen über 10 Gew.-% bieten keinen weiteren Vorteil, sind jedoch wiederum kostenintensiv und können die Aufarbeitung erschweren. Im allgemeinen werden die erfindungsgemäßen Co-Katalysatoren daher in Mengen von 0,01 — 5 Gew.-%, bevorzugt 0,05 — 2,5 Gew.-%, besonders bevorzugt 0,1 — 1 Gew.-%, jeweils bezogen auf das eingesetzte 4-Nitroalkylbenzol, eingesetzt.

Das Molverhältnis der eingesetzten Friedel-Crafts-Katalysatoren zu den Co-Katalysatoren kann im erfindungsgemäßen Verfahren ebenfalls in weiten Grenzen variiert werden. Im allgemeinen ist es vorteilhaft, den Co-Katalysator weder in zu großen Überschuss noch in zu großen Unterschuss gegenüber dem Friedel-Crafts-Katalysator einzusetzen. Im allgemeinen wird daher ein molares Verhältnis von Friedel-Crafts-Katalysator zu Co-Katalysator von (50-1) : (1-10), bevorzugt (10-1) : (1-5), besonders bevorzugt (3-1) : (1-2,5) gewählt.

Für die praktische Durchführung des erfindungsgemäßen Verfahrens ist die Reihenfolge der Zugabe der einzelnen Komponenten des Reaktionsgemisches beliebig. Hierbei lässt sich das Verfahren sowohl kontinuierlich als auch diskontinuierlich durchführen. Eine beispielhafte Ausführungsform ist die folgende:

Das gewünschte 4-Nitroalkylbenzol, beispielsweise 4-Nitrotoluol, wird vorgelegt und beispielsweise auf 90°C erwärmt. Dann gibt man in beliebiger Reihenfolge die gewünschten Mengen an Friedel-Crafts-Katalysator(en) und Co-Katalysator(en) zu und leitet unter weitgehender Konstanthaltung der Temperatur Chlor gasförmig in der vorgewählten Menge ein. Anschließend wird das Gemisch in üblicher Weise, etwa durch Destillation aufgearbeitet.

Eine weitere beispielhafte Ausführungsform ist die Folgende:

Man stellt eine Lösung von Katalysator und Co-Katalysator in dem gewünschten 4-Nitro-alkylbenzol her und

bringt diese auf die gewünschte Reaktionstemperatur. Dann wird ein Chlorierungsmittel in der vorgesehenen Menge zugesetzt. Auch hier kann die Aufarbeitung durch Destillation erfolgen.

Das erfindungsgemäße Verfahren ist gekennzeichnet durch eine außerordentlich hohe Selektivität für die gewünschte Monochlorstufe. Es erlaubt die Gewinnung von Chloriergemischen mit bis zu 95 — 96 % Gehalt an 2-Chlor-4-nitrotoluol. Dies ist vor allem überraschend im Hinblick auf US-A-5,095,157, die als Co-Katalysatoren nur ganz bestimmte komplexe schwefelhaltige Heterocyclen beschreibt.

Die erfindungsgemäßen Diarylsulfide sind aber überraschenderweise ebenso wirksam, auf der anderen Seite aber wesentlich einfacher zugänglich. Das erfindungsgemäße Verfahren vermeidet die Nachteile von jodhaltigen Chloriergemischen vollständig.

## Beispiele

In den nachfolgenden Beispielen werden die folgenden Abkürzungen verwendet:

| NT | 4-Nitrotoluol |
|---|---|
| 2-CNT: | 2-Chlor-4-nitrotoluol |
| 3-CNT: | 3-Chlor-4-nitrotoluol |
| DiCNT: | Summe der drei möglichen Dichlor-4-nitrotoluole |
| Rest: | Summe aller übrigen Komponenten |

Die Selektivität für die Bildung des 2-Chlor-4-nitroalkylbenzols wird wie folgt definiert:

$$\text{Selektivität S} = \frac{\text{Gehalt an 2-CNT x 100}}{\text{100-Restgehalt an 4-NT}}$$

## Vergleichsbeispiel 1 (kein Co-Katalysator)

In einem abgedunkelten schlanken Chlorierbecher werden 315 g 4-Nitrotoluol und 1,89 g wasserfreies $FeCl_3$ vorgelegt und bei 90°C unter Rühren 231 g Chlor gleichmäßig schnell in 7,5 Stunden eingeleitet. In festgelegten Zeitabständen werden Proben aus dem Reaktionsgemisch entnommen und durch Gaschromatographie untersucht. Die Ergebnisse sind in folgender Tabelle zusammengefasst:

| $Cl_2$ [1)] mol% | NT % | 2-CNT % | 3-CNT % | DiCNT % | Rest % | Selektivität 2-CNT |
|---|---|---|---|---|---|---|
| 39,8 | 63,89 | 35,07 | 0.34 | 0,27 | 0,52 | 97,12 |
| 76,6 | 32,33 | 65,28 | 0,50 | 1,50 | 0,41 | 96,47 |
| 89,4 | 21,72 | 74,98 | 0,50 | 2,38 | 0,42 | 95,78 |
| 108,4 | 9,02 | 85,70 | 0,42 | 4,38 | 0,38 | 94,19 |
| 119,4 | 2,48 | 89,05 | 0,27 | 7,70 | 0,50 | 91,31 |
| 130,4 | 0,19 | 85,17 | 0,09 | 13,82 | 0,73 | 85,33 |
| 141,6 | 0,02 | 76,91 | 0,01 | 21,89 | 1,17 | 76,92 |

[1)] eindosierte Chlormenge in mol % bezogen auf die eingesetzte Menge an 4-Nitrotoluol

## Vergleichsbeispiel 2 (Jod als Co-Katalysator)

In einem abgedunkelten Chlorierbecher werden 315 g 4-Nitrotoluol, 1,89 g wasserfreies $FeCl_3$ und 94,5 mg

Jod vorgelegt und auf 70°C erhitzt. Unter Rühren leitet man im Verlaufe von 7 Stunden 186 g Chlor ein. In vorher festgelegten Zeitabständen werden Proben gezogen und mittels GC analysiert. Die Ergebnisse sind in folgender Tabelle zusammengefasst:

| $Cl_2$ [1)] mol% | NT | 2-CNT | 3-CNT | DiCNT | Rest | Selektivität 2-CNT |
|---|---|---|---|---|---|---|
| 39,8 | 64,46 | 34,83 | 0,16 | 0,11 | 0,42 | 99,00 |
| 77,2 | 31,10 | 67,64 | 0,23 | 0,73 | 0,30 | 98,17 |
| 90,6 | 18,70 | 79,58 | 0,21 | 1,22 | 0,29 | 97,88 |
| 108,4 | 4,50 | 92,41 | 0,12 | 2,67 | 0,30 | 96,76 |
| 114,5 | 1,28 | 94,85 | 0,06 | 3,50 | 0,30 | 96,07 |

[1)] eindosierte Chlormenge in mol % bezogen auf die eingesetzte Menge an 4-Nitrotoluol

Nach der Chloreinleitung wird das Gemisch 30 Min. mit $N_2$ ausgeblasen, dann 3 x mit je 125 g Wasser bei ca. 70°C gewaschen. Man erhält 393,3 g an organischer leicht feuchter Produktphase mit einem 2-Chlor-4-nitrotoluolgehalt von 94,85 GC-Flächenprozent. Dieses Produkt wird auf Jod analysiert. Es hat einen Gesamtjodgehalt von 220 ppm.
Die Bedeutung der Abkürzungen in der Tabelle ist identisch zu Vergleichsbeispiel 1.

**<u>Beispiel 3</u> (Diphenylsulfid als Co-Katalysator)**

In einem abgedunkelten Chlorierbecher werden 407 g 4-Nitrotoluol, 3,1 g wasserfreies $FeCl_3$ und 3,1 g Diphenylsulfid vorgelegt und bei 90°C unter Rühren mit 242 g Chlor gleichmäßig schnell im Verlaufe von 3,75 Stunden mit Chlor begast. In festgelegten Zeitabständen wurden Proben aus dem Reaktionsgemisch entnommen und mittels GC untersucht. Die Ergebnisse sind in folgender Tabelle zusammengefasst:

| $Cl_2$ [1)] mol% | NT | 2-CNT | 3-CNT | DiCNT | Rest | Selektivität 2-CNT |
|---|---|---|---|---|---|---|
| 55,8 | 47,24 | 52,02 | 0,07 | 0,17 | 0,50 | 98,60 |
| 71,2 | 31,94 | 66,94 | 0,08 | 0,32 | 0,72 | 98,35 |
| 103,1 | 4,79 | 93,22 | 0,05 | 1,41 | 0,53 | 97,91 |
| 104,4 | 1,51 | 95,66 | 0,03 | 2,17 | 0,73 | 97,03 |
| 111,0 | 0,07 | 94,30 | 0,01 | 4,83 | 0,79 | 94,37 |
| 114,9 | 0,03 | 92,16 | 0,01 | 6,87 | 0,93 | 92,19 |

[1)] eindosierte Chlormenge in mol % bezogen auf die eingesetzte Menge an 4-Nitrotoluol

**<u>Beispiel 4</u> (4,4'-Dibromdiphenylsulfid als Co-Katalysator)**

In einem abgedunkelten Chlorierbecher werden 291 g 4-Nitrotoluol, 2,2 g wasserfreies $FeCl_3$ und 4,1 g 4,4'-Dibromdiphenylsulfid vorgelegt, unter Rühren auf 90°C erhitzt und bei dieser Temperatur im Verlaufe von 4 Stunden mit 196 g Chlor gleichmäßig schnell begast. In vorher festgelegten Zeitabständen werden Proben gezogen und mittels GC analysiert. Die Ergebnisse sind in folgender Tabelle zusammengefasst:

| $Cl_2$ [1)] mol% | NT | 2-CNT | 3-CNT | DiCNT | Rest | Selektivität 2-CNT |
|---|---|---|---|---|---|---|
| 98,2 | 16,07 | 82,02 | 0,09 | 0,85 | 0,91 | 97,80 |
| 104,8 | 10,84 | 87,03 | 0,08 | 1,12 | 0,93 | 97,61 |
| 110,8 | 7,09 | 90,43 | 0,07 | 1,45 | 0,96 | 97,33 |
| 114,8 | 5,24 | 91,99 | 0,66 | 1,83 | 0,88 | 97,08 |
| 120,1 | 2,15 | 94,28 | 0,04 | 2,55 | 0,98 | 96,35 |
| 124,7 | 1,02 | 94,59 | 0,03 | 3,33 | 1,03 | 95,56 |
| 130,0 | 0,60 | 93,79 | 0,02 | 4,33 | 1,06 | 94,36 |

[1)] eindosierte Chlormenge in mol % bezogen auf die eingesetzte Menge an 4-Nitrotoluol

Nach dem Ende der Chloreinleitung wird bei 90°C l h mit Stickstoff ausgeblasen. Nach dem Abkühlen erhält man eine Auswaage von 360,4 g. Berücksichtigt man einen Verlust von 4 g für die im Reaktionsverlauf entnommenen Proben, so entspricht das bei einem Endgehalt von 93,79 % 2-Chlor-4-nitrotoluol einer Ausbeute von rechnerisch 94,0 % der Theorie.

**<u>Beispiel 5</u> (4-Chlor-4'-Trifluormethyldiphenylsulfid als Co-Katalysator)**

Das Verfahren von Beispiel 3 wird wiederholt, nur dass statt des dortigen Co-Katalysators 4,75 g 4-Chlor-4'-Trifluormethyldiphenylsulfid eingesetzt wird und dass 253 g Chlor in 3,5 Stunden eingeleitet wird. Das Ergebnis ist in der folgenden Tabelle zusammengefasst:

| $Cl_2$ [1)] mol% | NT | 2-CNT | 3-CNT | DiCNT | Rest | Selektivität 2-CNT |
|---|---|---|---|---|---|---|
| 90,2 | 15,44 | 85,29 | 0,08 | 0,80 | 1,09 | 97,67 |
| 100,1 | 7,01 | 90,36 | 0,07 | 1,48 | 1,08 | 97,17 |
| 104,9 | 3,48 | 93,15 | 0,05 | 2,22 | 1,10 | 96,51 |
| 110,1 | 0,84 | 94,09 | 0,03 | 3,75 | 1,29 | 94,89 |
| 114,8 | 0,14 | 92,02 | 0,01 | 6,65 | 1,18 | 92,15 |
| 120,1 | 0,06 | 87,68 | 0,01 | 10,89 | 1,36 | 87,73 |

1) eindosierte Chlormenge in mol % bezogen auf die eingesetzte Menge an 4-Nitrotoluol

**<u>Beispiel 6</u> (4,4'-Dicyanodiphenylsulfid als Co-Katalysator)**

Das Verfahren von Beispiel 3 wird wiederholt, nur dass statt des dortigen Co-Katalysators 5,20 g 4,4'-Dicyanodiphenylsulfid und statt 3,1 g hier 4,1 g wasserfreies $FeCl_3$ eingesetzt werden. Bei 90°C wird in 4,5 Stunden 275 g Chlor eingeleitet. Das Ergebnis ist in der folgenden Tabelle zusammengefasst:

| $Cl_2$ [1] mol% | NT | 2-CNT | 3-CNT | DiCNT | Rest | Selektivität 2-CNT |
|---|---|---|---|---|---|---|
| 100,6 | 20,06 | 77,74 | 0,09 | 0,71 | 1,40 | 97,25 |
| 110,6 | 13,41 | 84,08 | 0,08 | 0,95 | 1,48 | 97,10 |
| 120,0 | 7,76 | 89,40 | 0,07 | 1,24 | 1,52 | 96,92 |
| 125,3 | 5,11 | 91,83 | 0,06 | 1,51 | 1,49 | 96,78 |
| 130,5 | 2,80 | 93,97 | 0,05 | 1,86 | 1,50 | 96,49 |

[1] eindosierte Chlormenge in mol % bezogen auf die eingesetzte Menge an 4-Nitrotoluol

Nach dem Ausblasen erhält man eine Auswaage von 503,5 g. Unter Berücksichtigung der Proben von ca. 3 g und dem Gehalt von 93,79 % ergibt sich eine Ausbeute von 93,3 % der Theorie an 2-Chlor-4-nitrotoluol.

**<u>Beispiel 7</u> (4,4'-Dihydroxydiphenylsulfid als Co-Katalysator)**

Das Verfahren von Beispiel 3 wird wiederholt, nur wird anstelle des dort verwendeten Co-Katalysator 3,6 g 4,4'-Dihydroxydiphenylsulfid eingesetzt. Bei 90°C wird in 4,5 Stunden 295 g Chlor eingeleitet. Das Ergebnis ist in der folgenden Tabelle zusammengefasst:

| $Cl_2$ [1] mol% | NT | 2-CNT | 3-CNT | DiCNT | Rest | Selektivität 2-CNT |
|---|---|---|---|---|---|---|
| 100,1 | 23,34 | 73,96 | 0,26 | 1,93 | 0,51 | 96,48 |
| 110,6 | 16,07 | 80,57 | 0,25 | 2,56 | 0,55 | 96,00 |
| 120,5 | 9,79 | 85,92 | 0,23 | 3,46 | 0,60 | 95,24 |
| 130,0 | 5,32 | 89,18 | 0,20 | 4,63 | 0,67 | 94,19 |
| 140,0 | 1,93 | 90,44 | 0,14 | 6,74 | 0,57 | 92,22 |

[1] eindosierte Chlormenge in mol % bezogen auf die eingesetzte Menge an 4-Nitrotoluol

**Patentansprüche**

**1.** Verfahren zur Herstellung von 2-Chlor-4-nitroalkylbenzolen der Formel I

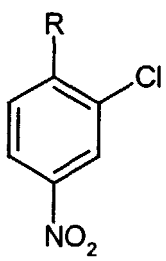

(I)

in der R für geradkettiges oder verzweigtes $C_1$-$C_4$-Alkyl steht,
durch Kernchlorierung des zugrundeliegenden 4-Nitroalkylbenzols mit elementarem Chlor oder Chlor abgebenden Verbindungen in flüssiger Phase in Gegenwart eines Friedel-Crafts-Katalysators und eines schwefelhaltigen aromatischen Co-Katalysators, dadurch gekennzeichnet, dass als schwefelhaltige aromatische Co-Katalysatoren Diarylsulfide der Formel

$$Ar^1 — S — Ar^2$$

eingesetzt werden, worin
$Ar^1$ und $Ar^2$ unabhängig voneinander Phenyl oder ein- oder mehrfach durch geradkettiges oder verzweigtes $C_1$-$C_8$-Alkyl, geradkettiges oder verzweigtes $C_1$-$C_8$-Halogenalkyl, geradkettiges oder verzweigtes $C_1$-$C_8$-Alkoxy, geradkettiges oder verzweigtes $C_1$-$C_8$-Alkylmercapto, Halogen, Cyano, Nitro, Hydroxy, Phenylmercapto oder Phenyl substituiertes Phenyl oder Naphthyl bedeuten.

**2.** Verfahren nach Anspruch 1, dadurch gekennzeichnet, dass als Verbindungen der Formel I 4-Nitrotoluol, 4-Nitroethylbenzol, 4-Nitropropylbenzol oder 4-Nitro-n-butylbenzol eingesetzt werden.

**3.** Verfahren nach Anspruch 1 oder 2, dadurch gekennzeichnet, dass als Co-Katalysatoren solche eingesetzt werden, bei denen $Ar^1$ und $Ar^2$ Phenyl oder ein- bis dreifach durch Fluor, Chlor oder Brom, geradkettiges oder verzweigtes $C_1$-$C_4$-Alkyl, $C_1$-$C_4$-Halogenalkyl, $C_1$-$C_4$-Alkylmercapto oder Phenyl substituiertes Phenyl bedeuten.

**4.** Verfahren nach einem oder mehreren der Ansprüche 1-3, dadurch gekennzeichnet, dass als Co-Katalysatoren solche eingesetzt werden, bei denen $Ar^1$ und $Ar^2$ Phenyl oder ein- oder zweifach durch Chlor, Brom oder Methyl substituiertes Phenyl bedeuten.

**5.** Verfahren nach einem oder mehreren der Ansprüche 1-4, dadurch gekennzeichnet, dass bei einer Reaktionstemperatur von 50 — 150°C, bevorzugt 70 — 120°C und besonders bevorzugt 80 — 100°C gearbeitet wird.

**6.** Verfahren nach einem oder mehreren der Ansprüche 1-5, dadurch gekennzeichnet, dass 90 — 130 Mol-%, bevorzugt 95 — 110 Mol-% Chlor in elementarer Form oder in Form einer Chlor abgebenden Substanz pro Mol 4-Nitroalkylbenzol eingesetzt werden.

**7.** Verfahren nach einem oder mehreren der Ansprüche 1-6, dadurch gekennzeichnet, dass als Friedel-Crafts-Katalysatoren Antimonchloride, Aluminiumchlorid oder Eisenchloride, bevorzugt Eisen-(III)-chlorid, eingesetzt werden.

**8.** Verfahren nach einem oder mehreren der Ansprüche 1-7, dadurch gekennzeichnet, dass der Friedel-Crafts-Katalysator oder ein Gemisch mehrerer von ihnen in einer Menge von 0,01 — 3 Gew.-%, bevorzugt von 0,05 — 1,5 Gew.-%, besonders bevorzugt von 0,1 — 1,00 Gew.-%, eingesetzt wird, jeweils bezogen auf die Menge des eingesetzten 4-Nitroalkylbenzols.

**9.** Verfahren nach einem oder mehreren der Ansprüche 1-8, dadurch gekennzeichnet, dass der Co-Katalysator oder ein Gemisch mehrerer von ihnen in einer Menge von 0,01 — 5 Gew.-%, bevorzugt 0,05 — 2,5 Gew.-%, besonders bevorzugt 0,1 — 1 Gew.-%, jeweils bezogen auf die Menge des eingesetzten 4-Nitroalkylbenzols, eingesetzt wird.

**10.** Verfahren nach einem oder mehreren der Ansprüche 1-9, dadurch gekennzeichnet, dass das molare Verhältnis von Friedel-Crafts-Katalysator zu Co-Katalysator von (50-1) : (1-10), bevorzugt (10-1) : (1-5), besonders bevorzugt (3-1) : (1-2,5) beträgt.

Europäisches Patentamt

# EUROPÄISCHER RECHERCHENBERICHT

Nummer der Anmeldung
EP 00 11 7274

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,Y | EP 0 071 038 A (BAYER AG)<br>9. Februar 1983 (1983-02-09)<br>* Ansprüche *<br>--- | 1-10 | C07C205/12<br>C07C201/12 |
| D,Y | EP 0 399 293 A (BAYER AG)<br>28. November 1990 (1990-11-28)<br>* das ganze Dokument *<br>--- | 1-10 | |
| Y | EP 0 150 587 A (ICI PLC)<br>7. August 1985 (1985-08-07)<br>* Ansprüche *<br>--- | 1-10 | |
| Y | US 3 920 757 A (WATSON WILLIAM DAVID)<br>18. November 1975 (1975-11-18)<br>* Ansprüche *<br>----- | 1-10 | |

RECHERCHIERTE SACHGEBIETE (Int.Cl.7)
C07C

Der vorliegende Recherchenbericht wurde für alle Patentansprüche erstellt

| Recherchenort | Abschlußdatum der Recherche | Prüfer |
|---|---|---|
| DEN HAAG | 11. Dezember 2000 | Bonnevalle, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03 82 (P04C03)

**ANHANG ZUM EUROPÄISCHEN RECHERCHENBERICHT ÜBER DIE EUROPÄISCHE PATENTANMELDUNG NR.** EP 00 11 7274

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-12-2000

| Im Recherchenbericht angeführtes Patentdokument | Datum der Veröffentlichung | Mitglied(er) der Patentfamilie | Datum der Veröffentlichung |
|---|---|---|---|
| EP 0071038 A | 09-02-1983 | DE 3128442 A | 03-02-1983 |
| | | DE 3260205 D | 12-07-1984 |
| | | US 4456777 A | 26-06-1984 |
| EP 0399293 A | 28-11-1990 | DE 3916664 A | 03-01-1991 |
| | | DD 297957 A | 30-01-1992 |
| | | DE 59002525 D | 07-10-1993 |
| | | JP 3020244 A | 29-01-1991 |
| | | US 5095157 A | 10-03-1992 |
| EP 0150587 A | 07-08-1985 | AT 32333 T | 15-02-1988 |
| | | AU 571039 B | 31-03-1988 |
| | | AU 3683784 A | 18-07-1985 |
| | | BR 8500073 A | 20-08-1985 |
| | | CA 1289974 A | 01-10-1991 |
| | | DE 3469167 D | 10-03-1988 |
| | | GB 2154581 A,B | 11-09-1985 |
| | | HU 36769 A,B | 28-10-1985 |
| | | JP 1927182 C | 25-04-1995 |
| | | JP 6057680 B | 03-08-1994 |
| | | JP 60158142 A | 19-08-1985 |
| | | NZ 210507 A | 20-02-1987 |
| US 3920757 A | 18-11-1975 | AU 482414 B | 21-10-1976 |
| | | BR 7502276 A | 05-10-1976 |
| | | CA 1032959 A | 13-06-1978 |
| | | DE 2516567 A | 21-10-1976 |
| | | FR 2307785 A | 12-11-1976 |
| | | GB 1476036 A | 10-06-1977 |
| | | JP 1227956 C | 19-09-1984 |
| | | JP 51122031 A | 25-10-1976 |
| | | JP 58058329 B | 24-12-1983 |
| | | NL 7504410 A,B, | 18-10-1976 |
| | | SE 403610 B | 28-08-1978 |
| | | AU 8013975 A | 21-10-1976 |
| | | BE 827912 A | 14-10-1975 |
| | | SE 7504345 A | 16-10-1976 |

EPO FORM P0461

Für nähere Einzelheiten zu diesem Anhang : siehe Amtsblatt des Europäischen Patentamts, Nr.12/82